Europäisches Patentamt

European Patent Office

Office européen des brevets

(19)

(11) **EP 0 561 145 B1**

(12) **EUROPÄISCHE PATENTSCHRIFT**

(45) Veröffentlichungstag und Bekanntmachung des Hinweises auf die Patenterteilung:
**03.12.1997 Patentblatt 1997/49**

(51) Int. Cl.$^6$: **A61K 33/40**

(21) Anmeldenummer: **93101888.1**

(22) Anmeldetag: **06.02.1993**

(54) **Verwendung einer chemisch stabilisierten Chloritmatrix zur Herstellung von Arzneimitteln zur Behandlung von HIV-Infektionen**

Use of a chemicallly stabilized chlorite matrix for the manufacture of medicaments for the treatment of HIV infections

Utilisation d'une matrice faite à base de chlorite pour la manufacture de préparations médicinales pour le traitement d'infections causées par le HIV

(84) Benannte Vertragsstaaten:
**AT BE CH DE DK ES FR GB GR IE IT LI LU MC NL PT SE**

(30) Priorität: **19.03.1992 DE 4208828**

(43) Veröffentlichungstag der Anmeldung:
**22.09.1993 Patentblatt 1993/38**

(73) Patentinhaber:
• **OXO Chemie AG**
**1701 Fribourg (CH)**
Benannte Vertragsstaaten:
**BE CH DK ES FR GB GR IE IT LI LU MC NL PT SE AT**
• **OXO Chemie GmbH**
**39164 Wanzleben (DE)**
Benannte Vertragsstaaten:
**DE**

(72) Erfinder:
**Kühne, Friedrich W., Dr.**
**W-6900 Heidelberg (DE)**

(74) Vertreter:
**Grussdorf, Jürgen, Dr. et al**
**Patentanwälte Zellentin & Partner**
**Rubensstrasse 30**
**67061 Ludwigshafen (DE)**

(56) Entgegenhaltungen:
| | |
|---|---|
| EP-A- 0 200 155 | EP-A- 0 200 156 |
| EP-A- 0 200 157 | WO-A-89/03179 |
| WO-A-89/10747 | WO-A-90/01315 |
| US-A- 4 296 103 | US-A- 5 019 402 |

**Beschreibung**

Gegenstand der vorliegenden Erfindung ist die neue Verwendung einer chemisch stabilisierten Chloritmatrix zur Herstellung von Arzneimitteln in Form einer isotonischen Lösung zur Behandlung von HIV-Infektionen.

HIV-Infektionen, worunter im folgenden die verschiedenen Unterformen des HIV-Virus verstanden werden sollen, sind nach den vorliegenden Statistiken im Vormarsch. Trotz intensiver Bemühungen der Fachwelt ist es bisher nicht gelungen, ein absolut wirksames Gegenmittel zu finden. Bisher bekannte Mittel wie AZT oder DDI wirken insbesondere auf die mit dem Virus infizierten Zellen, die dadurch abgetötet werden, so daß die Infektion nicht weiterläuft, haben jedoch keinen Effekt auf die durch das Aufbrechen solcher Zellen in die Blutbahn freigesetzten Viren, welche die Verbreitung und Neuinfektion anderer Zellen bewirken.

Aufgabe der vorliegenden Erfindung ist es daher, ein Mittel zu finden, welches HIV-Viren im Blut inaktiviert, ohne jedoch auf das Blut und den Körper des Patienten einen schädigenden Einfluß zu haben.

Überraschenderweise wird diese Aufgabe durch die im Hauptanspruch beschriebenen Merkmale gelöst und durch die in den Unteransprüchen gekennzeichneten Merkmale gefördert.

Die vorliegende Erfindung betrifft daher die Verwendung einer chemisch stabilisierten Chloritmatrix, bestehend aus einer isotonischen Lösung mit einem Gehalt von 5 - 100 mMol $ClO_2^-/\ell$ Lösung, zur Herstellung von Arzneimitteln zur Behandlung von HIV-Injektionen.

Es ist bekannt, daß Lösungen von Natrium- oder Kaliumchlorit in saurer Lösung unter Bildung von Chlordioxid und Chlorid disproportionieren. Die Chlorite und auch das gebildete Chlordioxid sind als wirksame Mikrobiozide bei der Sterilisierung von Oberflächen und Lösungen bekannt. Die Verwendungen von Chloritlösungen bei einer parenteralen Applikation verbietet sich jedoch, weil diese außerordentlich toxisch sind.

Aus Sarin et al., New England Journal of Medicine, 313, 1416 (1985) ist es bekannt, daß eine Behandlung von Zellkulturen von HTLV III - (HIV) Viren in vitro mit einer 200-fach verdünnten Lösung von 0,23 % Natriumchlorit und 1,26 % Milchsäure zu einer Inaktivierung der Viren führte.

Aus USP 5 019 402 ist weiterhin bekannt, daß eine Lösung, die Chlordioxid oder eine Chlordioxid freisetzende Mischung aus einem Chlorit, einem schwach sauren Puffer und einem hitzeaktivierten Saccharid enthält, zur Sterilisierung von Blutkonserven mit Ausnahme von solchen, die rote Blutkörperchen enthalten, d.h. Leucozyten, Blutplättchen und Koagulationsfaktoren und Globuline, verwendet werden können. In Vollblut tritt eine entsprechende desinfizierende Wirkung nicht ein, offensichtlich weil die roten Blutkörperchen schneller von dem Chlordioxid angegriffen werden als die untersuchten Mikroorganismen. Auch dieses Mittel ist daher für eine parenterale Applikation nicht geeignet.

Aus der DE-OS 3 213 389 sowie der USP 4 296 103 sind chemisch stabilisierte Chloritmatices bekannt geworden, welche für eine äußerliche oder orale therapeutische Verwendung geeignet sind. Neben verschiedenen bakteriellen Infektionen wird auch die äußerliche Behandlung von Virusinfektionen wie Herpes simplex und Herpes zoster auf diese Weise für möglich erachtet. Eine intravenöse Applikation zur Behandlung von HIV-Infektionen ist auf diesem Wege nicht möglich.

Aus der EP 0 200 155 sind ferner wässrige Lösungen einer chemisch stabilisierten Chloritmatrix zur intravenösen und perioperativen Verabreichung bekannt. Das Mittel erwies sich bei Candida Albicans-Infektionen als wirksam.

Aus der EP 0 200 157 ist bekannt, solche stabiliserte Chloritmatrices zur intravenösen und/oder lokalen Applikation bei infektiösen Zuständen, hervorgerufen durch Parasiten, Pilze, Bakterien, Viren und/oder Mykoplasten, zu verwenden. Die Wirkung wird durch eine Phagozytenstimulierung erklärt, die durch eine einmalige, kurz nach der Infektion wirkende Gabe des Chloritkomplexes erreicht wird. Eine Bekämpfung von Virusinfektionen ist in dieser Schrift beispielhaft nicht beschrieben und erscheint wegen des Wirkprinzips auch nicht möglich.

Es ist daher außerordentlich überraschend, daß man mit einer intravenösen Applikation einer geeigneten Chloritmatrix, wie sie beispielsweise gemäß der DE-OS 3 213 389 und USP 4 296 103 hergestellt wurde, in geeigneter Konzentration HIV-Viren direkt im Blut bekämpfen kann, was durch den raschen und starken Abfall der im Blut nachweisbaren Viren belegt wird. Die Viruskonzentration im Serum wurde in bekannter Weise durch Kopplung mit Antikörpern gegen das virusspezifische p24-Antigen bestimmt.

Die Chloritmatrix gemäß DE-OS-3 213 389 wird durch Oxidation einer Chloritlösung mit Hypochlorit, und Umsetzung mit Perborat oder Percarbonat hergestellt.

Erwartungsgemäß erweist sich die Wirkung als konzentrationsabhängig. Eine signifikante Inhibierung der Neuinfektion wird in vitro bereits bei Konzentrationen von 5 µMol/l gefunden, während eine Konzentration von 150 µMol/l praktisch eine vollständige Inhibierung bewirkt. Da Konzentrationen von über 100 µMol/l auf die Dauer jedoch zu cytotoxischen Schäden führen können, werden Konzentrationen zwischen 10 und 100, vorzugsweise etwa 40 bis 80 und insbesondere 50 µMol/l, bevorzugt. In vivo ist entsprechend dem Körpergewicht zu dosieren, wobei wegen des laufenden Abbaus des Wirkstoffs im Blut in regelmäßigen Abständen das Mittel erneut verabreicht werden muß.

**Versuchsprotokoll**

Die in den folgenden Versuchen als WF10 bezeichnete Chloritmatrix wurde gemäß DE 32 13 389, Beispiel 1, hergestellt und mit einer isotonischen Lösung von Kochsalz bzw. dem entsprechenden Nährmedium auf die in den Versuchen angegebenen Konzentrationen verdünnt.

### In vitro - Versuche

Um die Wirksamkeit von WF10 auf die HIV-Vermehrung in menschlichen Zell-Linien zu untersuchen, wurde die Infektivität (TCID50), die Aktivität der reversen Transkriptase (RT) und die Bildung von HIV-Antigen p24 untersucht.

### Verfahren

Permanent durch HIV-1 infizierte menschliche T-Lymphomazellen (Molt-4, Clon-8-Zellen) werden mit RPMI-1640 Medium, welches mit 10 % FCS, L-Glutaminsäure und Antibiotika versetzt ist, kultiviert. In gleicher Weise werden die menschliche monozyte Makrophagen-Zellinie U937 und primäre einkernige Blutzellen (PWMC), welche mit HIV-1 permanent infiziert sind, untersucht.

Zur Messung der Aktivität der HIV-Reverstranskriptase wird 1 ml des Kulturüberstandes mit einem Standard-Enzym-Untersuchungssystem, welches auf dem Einbau radioaktiv markierter Nucleotide beruht, untersucht. Diese Bestimmung gibt einen Hinweis auf die mögliche Auswirkung der Substanz auf die Ausbreitung der HIV-Infektion.

Die Konzentration des HIV-Kernantigens p24 wird im Überstand durch Elisatechniken mit einem handelsüblichen Kit (Abbot) untersucht, der einen internen Standard enthält. Die Konzentrationen in den folgenden Tabellen sind in ng/ml Kulturflüssigkeit angegeben. In dem Test wird dabei nicht zwischen p24 Antigen aus infektiösen oder inaktivierten HIV-Viren unterschieden.

Die Infektivität des HIV (TCID50) wird bestimmt, indem man die HIV-haltige Lösung mit den hochempfindlichen MT4-Zellen (American Tissue Collection) zusammenbringt, die durch infektiöse HIV-1 innerhalb kurzer Zeiten abgetötet werden, was sich durch das ausbleibende Wachstum der Zellkolonie zeigt. Der cytopathische Effekt kann ferner durch die Einbaurate von $^3$H-Thymidin bestimmt werden, da abgetötete Zellen kein Thymidin mehr in ihre DNA einbauen und somit aus der Stärke des Thymidin-Einhaus bzw. der dadurch bewirkten Radioaktivität der von der Lösung separierten Zell-DNA auf die Menge der Überlebenden Zellen geschlossen werden kann.

Da diese Untersuchungen in der Technik gut bekannt sind, wird auf eine nähere Beschreibung verzichtet.

### Vermehrung von HIV in Gegenwart von WF10

$2 \times 10^6$ Molt4/8-Zellen, welche permanent mit HIV-1 infiziert sind, werden in 2 ml Kulturmedium 24 Stunden in Gegenwart verschiedener Konzentrationen von WF10 mit und ohne Zugabe von Hämoglobin, auf einer 24-Napfkulturplatte inkubiert (Tabelle 1).

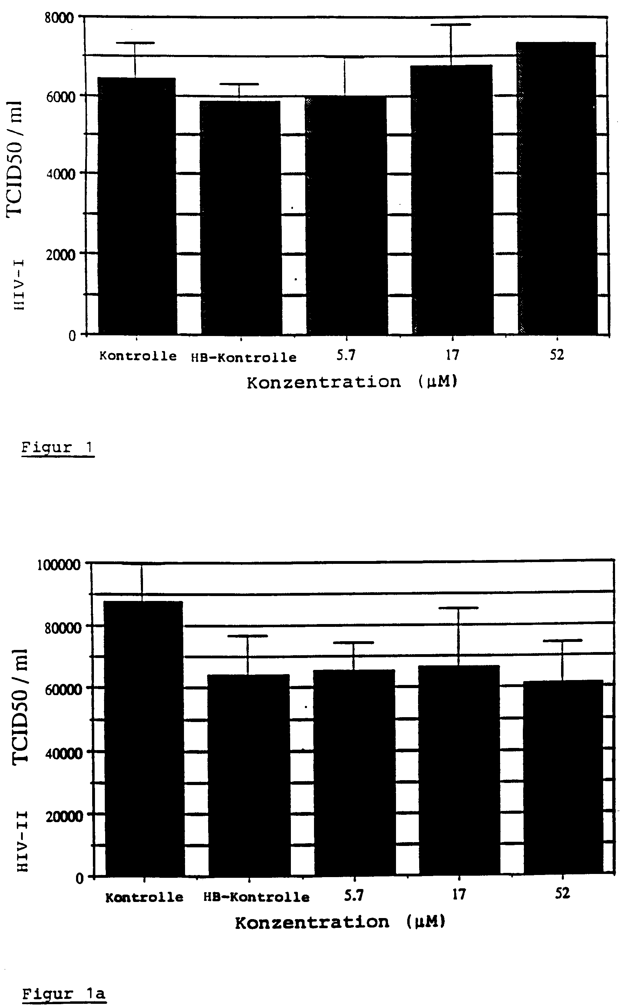

Aus den Figuren 1 und 1a lassen sich entnehmen, daß für die untersuchten Konzentrationen von 5,7 bis 52 µMol die TCID50/l nicht signifikant von der Kontrolle unterschieden ist. WF10 hat demgemäß keinen Effekt auf zellgebundene HIV-I oder HIV-II.

### Infektivität von ungebundenem HIV nach Inkubation mit WF10

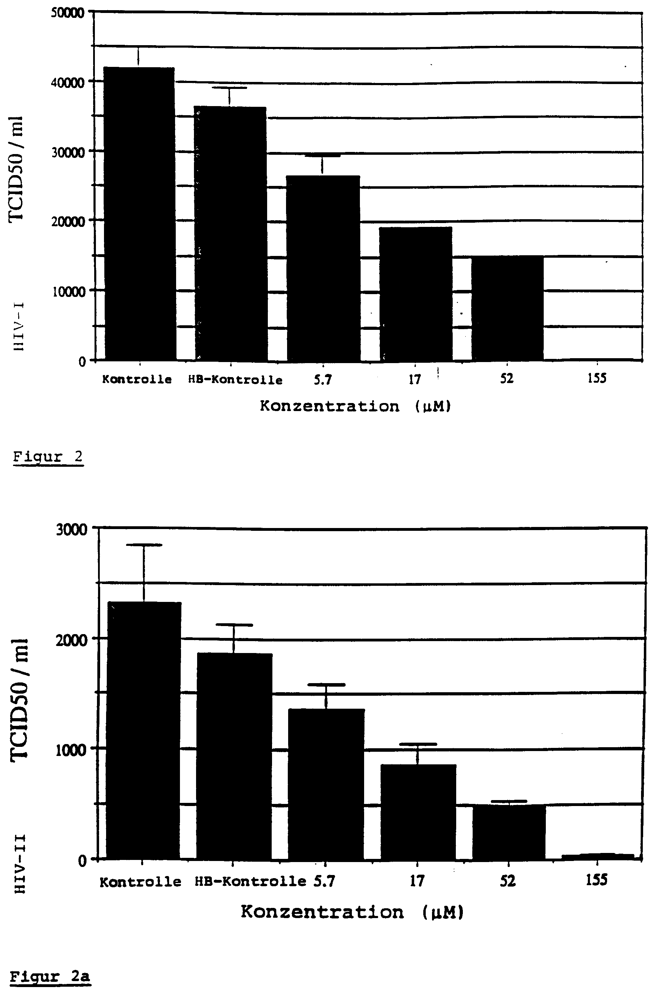

Um die mögliche Inaktivierung durch WF10 auf freie HIV-Partikel zu untersuchen, wurden die in der Tabelle 2 wiedergegebenen WF10-Verdünnungen und -Kontrollösungen mit einer entsprechenden HIV-Stammlösung während der angegebenen Zeiten inkubiert. Die Ergebnisse sind in den Figuren 2 und 2a wiedergegeben.

Es zeigt sich, daß WF10 in der untersuchten Konzentration HIV-Viren wirksam inhibiert.

### Neuinfektion von Zellen mit HIV in Gegenwart von WF10

In einem weiteren Experiment wurde die Ausbreitung des Virus in Zellkulturen nach Infektion mit HIV bestimmt. Kulturen von Molt4/8-Zellen werden mit HIV-1-Stammlösung in verschiedenen Verdünnungen infiziert. Das Kulturmedium wird nach jeweils 2 Tagen ausgetauscht und im Überstand die Infektivität mit MT4-Zellen bestimmt. Soweit die Lösung einen Wirkstoff (WF10, AZT oder DDI oder Kombinationen davon) enthält, werden diese dem Kulturmedium in der entsprechenden Konzentration beim Austausch jeweils wieder zugesetzt.

### In vivo - Experimente

Die erfindungsgemäßen Mittel wurden auch bereits an einem kleinen Kollektiv von Patienten mit akuter HIV-Infektion getestet. Zu diesem Zweck wurden den Patienten 0,5 ml/kg Körpergewicht von WF10 mit einer Konzentration von 60 mMol/l $ClO_2^-$ jeweils an fünf aufeinanderfolgenden Tagen einer Woche injiziert, zwei Wochen pausiert, dann die p24-Antigen-Konzentration bestimmt und die Behandlung in gleichem Turnus noch ein- bis dreimal fortgeführt.

Die Ergebnisse sind in der Tabelle 3 wiedergegeben.

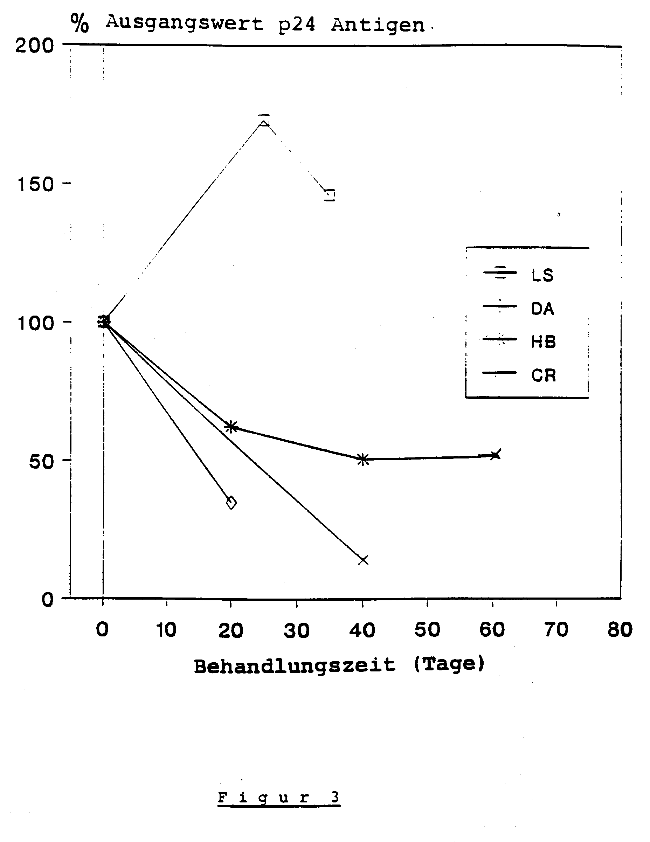

Der Einfluß auf den Serumspiegel von p24-Antigen bei 4 HIV-1-infizierten Patienten, die entsprechend mit WF10 behandelt wurden, ist in der Tabelle 3 sowie der Figur 3, bezogen auf den Wert vor der Behandlung als Basis = 100 %, wiedergegeben. Man sieht, daß in allen anderen Fällen eine sehr rasche, starke Abnahme zu bemerken ist, die auch über die längere Behandlungsdauer anhält. Eine Ausnahme bildet der der Patient LS, der einen ursprünglich sehr niedrigen Titer an p24-AG hatte, wobei der Titer zunächst ansteigt und erst nach dem 30. Tage wieder signifikant abfällt. Da der Titer des p24-Antigens ein Maß für die Konzentration der Viren im Serum ist, ist aus diesem Befund zu entnehmen, daß WF10 gerade die ungebundenen Viren im Serum angreift und inaktiviert und somit die Neuinfektion noch nicht geschädigter Zellen zu inhibieren vermag.

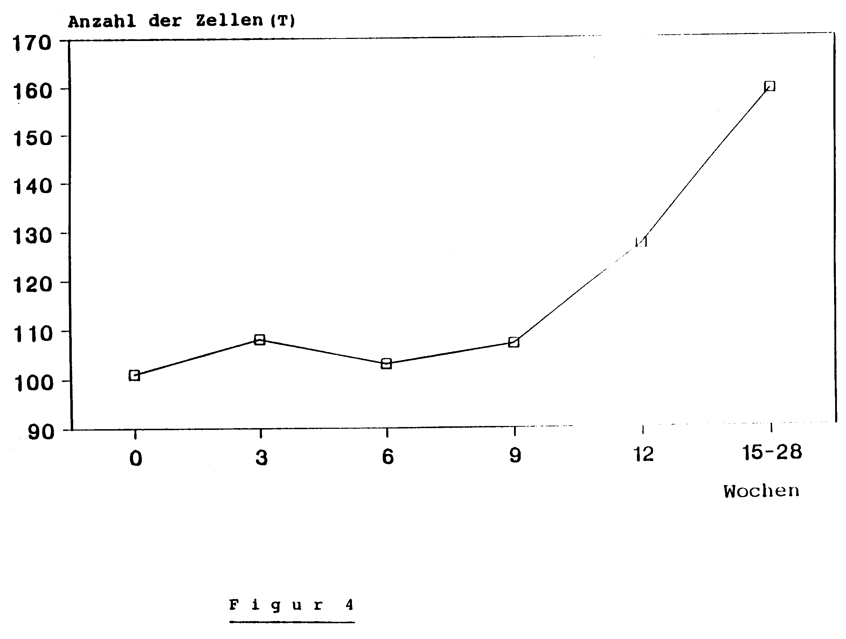

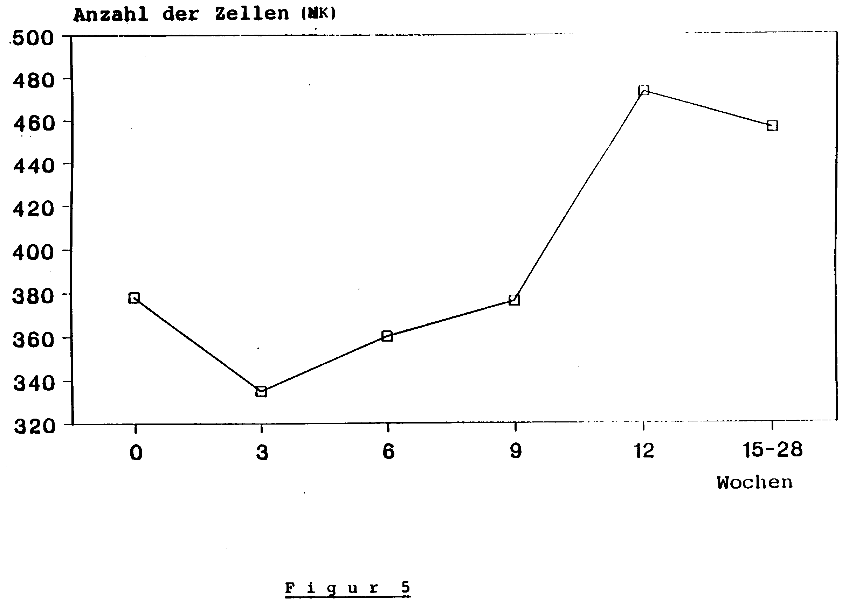

Ferner wurde die für die Immunabwehr des Körpers wichtige Konzentration der T-Zellen und NK-Zellen während der Behandlung mit WF10 bestimmt. Es zeigt sich, daß die Anzahl der für die Immunabwehr wichtigen Zellen nach einer Behandlungszeit von 9 Wochen anzusteigen beginnt, d.h. eine Langzeitimmunisierung durch WF10 stimuliert werden kann. Die bei Gabe von WF10 beobachtete kurzfristige Senkung der p24-Antigenkonzentration im Serum läßt sich jedoch aufgrund dieses Mechanismus nicht erklären, was ein weiterer Hinweis darauf ist, daß eine direkte Wechselwirkung von WF10 mit den freien, ungebundenen Viren besteht. Die Versuchsergebnisse sind in den Figuren 4 und 5 wiedergegeben.

Tabelle 1

| Behandlung von HIV-infizierten Zellen mit WF10 | | | |
|---|---|---|---|
| No. | Substanz | Konzentration | Zugabe |
| 1 | - | - | - |
| 2 | - | - | Hb[a] |
| 3 | WF10 | 1.9 μM | - |
| 4 | WF10 | 5.7 μM | - |
| 5 | WF10 | 17.2 μM | - |
| 6 | WF10 | 51.7 μM | - |
| 7 | WF10 | 1.9 μM | Hb |
| 8 | WF10 | 5.7 μM | Hb |
| 9 | WF10 | 17.2 μM | Hb |
| 10 | WF10 | 51.7 μM | Hb |

[a] Hb = Hämoglobin (250 μg/ml)

Tabelle 2

| Inaktivierung von HIV durch WF10 | | | | |
|---|---|---|---|---|
| No. | Substanz | Konzentration | Zugabe | Zeit |
| 1 | - | - | - | 30 min |
| 2 | - | - | Hb[a] | - |
| 3 | WF10 | 155 µM | - | - |
| 4 | WF10 | 155 µM | Hb | - |
| 5 | - | - | - | 1 hr |
| 6 | - | - | Hb | - |
| 7 | WF10 | 155 µM | - | - |
| 8 | WF10 | 155 µM | Hb | - |
| 9 | - | - | - | 3 hrs |
| 10 | - | - | Hb | - |
| 11 | WF10 | 155 µM | - | - |
| 12 | WF10 | 155 µM | Hb | - |
| 13 | - | - | - | 6 hrs |
| 14 | - | - | Hb | - |
| 15 | WF10 | 155 µM | - | - |
| 16 | WF10 | 155 µM | Hb | - |
| 17 | - | - | - | 18 hrs |
| 18 | - | - | Hb | - |
| 19 | WF10 | 155 µM | - | - |
| 20 | WF10 | 155 µM | Hb | - |

[a] Hb = Hämoglobin (250 µg/ml)

Tabelle 3
==========

p24 AG Konz. (pg/ml und % Ausgangswert) bei Behandlung mit WF10

| Patient | CR | % | DA | % | LS | % | HB | % |
|---|---|---|---|---|---|---|---|---|
| Datum | | | | | | | | |
| 0 | 666 | 100.0 | 483 | 100.0 | 173 | 100.0 | 5363 | 100.0 |
| 19 | – | – | 169 | 35.0 | – | – | – | – |
| 21 | – | – | – | – | – | – | 3353 | 62.5 |
| 28 | – | – | – | – | 300 | 173.4 | – | – |
| 35 | – | – | – | – | 253 | 146.2 | – | – |
| 39 | 95 | 14.3 | – | – | – | – | – | – |
| 42 | – | – | – | – | – | – | 2713 | 50.6 |
| 63 | – | – | – | – | – | – | 2603 | 48.5 |

**Patentansprüche**

1. Verwendung einer chemisch stabilisierten Chloritmatrix, bestehend aus einer isotonischen Lösung mit einem Gehalt von 5 - 100 mMol $ClO_2^-$/l Lösung, zur Herstellung von Arzneimitteln zur Behandlung von HIV-Infektionen.

2. Verwendung gemäß Anspruch 1, **dadurch gekennzeichnet**, daß die Lösung einen $ClO_2^-$-Gehalt an 50 - 80 mMol/l aufweist.

3. Verwendung gemäß Anspruch 1 oder 2, **dadurch gekennzeichnet**, daß die Lösung direkt injiziert oder in einem größeren Volumen einer Infusionslösung verdünnt wird.

4. Verwendung gemäß einem der Ansprüche 1 bis 3, **dadurch gekennzeichnet**, daß die Chloritmatrix durch Oxidation einer Chloritlösung mit Hypochlorit, und Umsetzung mit Perborat oder Percarbonat hergestellt ist.

**Claims**

1.  Use of a chemically-stabilised chlorite matrix consisting of an isotonic solution containing 5 to 100 mMol $ClO_2^-$ per litre of solution, for the production of medicines for the treatment of HIV infections.

2.  Use according to claim 1, wherein the solution has a $ClO_2^-$ content of 50 to 80 mMol/l.

3.  Use according to claim 1 or 2, wherein the solution is injected as such or diluted in a larger volume of an infusion.

4.  Use according to one of claims 1 to 3, wherein the chlorite matrix is produced by oxidation of a chlorite solution with hypochlorite and reaction with perborate or percarbonate.

**Revendications**

1.  Utilisation d'une matrice de chlorite stabilisée chimiquement, constituée d'une solution isotonique avec une teneur de 5 à 100 mmoles de $ClO_2^-$ par litre de solution, pour la manufacture de préparations médicinales pour le traitement d'infections causées par le HIV.

2.  Utilisation selon la revendication 1, caractérisée en ce que la solution présente une teneur en $ClO_2^-$ de 50 à 80 mmole/l.

3.  Utilisation selon la revendication 1 ou 2, caractérisée en ce que la solution est injectée directement ou diluée dans un volume plus important d'une solution de perfusion.

4.  Utilisation selon l'une des revendications 1 à 3, caractérisée en ce que la matrice de chlorite est préparée par oxydation d'une solution de chlorite avec de l'hypochlorite et par réaction avec du perborate ou du percarbonate.

Figur 1

Figur 1a

Figur 2

Figur 2a

Figur 3

EP 0 561 145 B1

**Anzahl der Zellen (T)**

Wochen

Figur 4

**Anzahl der Zellen (MK)**

F i g u r  5

EP 0 561 145 B1